# EUROPEAN PATENT APPLICATION

(11) **EP 3 848 063 A1**
(43) Date of publication of application: **14.07.2021**
(21) Application number: 19858511.9
(22) Date of filing: 02.09.2019
(51) Int. Cl.: A61L 27/22, A61L 27/18, A61L 27/26, A61L 27/38, A61L 27/52, C07K 14/78, C07K 17/04, C12N 5/07, C12N 5/077, C12N 5/0775, C12N 11/04, C12N 15/12

(54) **GEL FORMATION KIT, GEL, AND GEL PRODUCTION METHOD**

(30) Priority: 03.09.2018 JP 2018164644; 26.03.2019 JP 2019058680
(71) Applicant: FUJIFILM Corporation, Tokyo 106-8620 (JP)
(72) Inventor: KAMATA, Hiroyuki, Ashigarakami-gun, Kanagawa 258-8577 (JP); HIRATSUKA, Takahiro, Ashigarakami-gun, Kanagawa 258-8577 (JP); HADA, Satoko, Gamagori-shi, Aichi 443-0022 (JP); YANADA, Shinobu, Gamagori-shi, Aichi 443-0022 (JP)
(74) Representative: Hoffmann Eitle
(86) International application number: PCT/JP2019/034390
(87) International publication number: WO 2020/050205

(57) **Abstract**

An object of the present invention is to provide a gel forming kit capable of regenerating hyaline cartilage, a gel that is formed by using the gel forming kit, and a method for producing a gel using the gel forming kit. The present invention provides a gel forming kit containing a crosslinking agent having at least two chains each of which includes a functional group capable of being covalently bonded to an amino group and includes a hydrophilic linking group, and a recombinant peptide; a gel formed by crosslinking of a recombinant peptide with a crosslinking agent having at least two chains each of which includes a functional group capable of being covalently bonded to an amino group and includes a hydrophilic linking group; and a method for producing the gel.

## Description

### Field of the Invention

The present invention relates to a gel forming kit containing a crosslinking agent and a recombinant peptide. The present invention further relates to a gel formed by crosslinking a recombinant peptide with a crosslinking agent, and a method for producing the gel.

### Description of the Related Art

Articular cartilage plays a role as a cushion to release the impact. In a case where articular cartilage is damaged by excessive load or the like, it becomes painful and the quality of life is significantly deteriorated. Although cartilage is an important tissue, it does not naturally restore oneself once it is damaged.

Due to the insufficient self-restoring ability of cartilage, surgical procedures such as resection of the injured cartilage, bone marrow stimulation, and implantation of bone trabecula are usually required to promote the restoration of cartilage. Bone marrow stimulation is often used as the first choice because it induces stem cells and various growth factors inside the bone marrow to gather at the affected part by promoting bleeding from the bone marrow and can be performed in a minimally invasive manner under a microscope. However, the tissue regenerated by bone marrow stimulation is "fibrous cartilage" which has different histological and mechanical properties from "hyaline cartilage" which is the natural cartilage. In a case where regeneration occurs in fibrous cartilage, this tissue lacks durability, thereby eventually leading to knee osteoarthritis as reported in some cases. One of the causes of this is that the blood clot, which is composed of bone marrow components, decomposes at an early stage and a scaffold for cells is lost.

In order to make up for the shortcomings of bone marrow stimulation, it is conceivable to form a scaffold for cells in the affected part in a minimally invasive manner. Since a shape of a cartilage defective part varies, it is desirable that a scaffold matching the shape of the affected part is formed after the affected part is filled with the liquid component. It is important that a material to be injected has cell adhesiveness such that the material functions as a scaffold for cells, which plays a major role in tissue reconstruction. It is conceivable to use an injectable gel for this purpose. Attempts to gelate biocompatible polymers such as polyethylene glycol (PEG) are widely known, and such polymers are sometimes used as a biological sealant.

On the other hand, many synthetic polymers do not have cell adhesiveness, which is not preferable as a scaffold for tissue reconstruction. A collagen scaffolding material, which is sometimes used in incisional surgery, is usually insoluble in water, and thus it is difficult to firmly attach the collagen scaffolding material to the affected part at which the collagen scaffolding material is injected and to gelate the attached collagen scaffolding material. On the other hand, since collagen-derived gelatin is water-soluble, it is possible to form a gel in the desired form after injection, by using a crosslinking agent such as polyfunctional PEG.

Patent Document 1 discloses an implant containing a hydrogel which contains a first reactive precursor containing a multi-arm polyether having an electrophilic group; a second reactive precursor containing a nucleophilic group; and at least one initiating precursor containing at least one vinyl group.

Patent Document 2 discloses a tissue scaffold having a first hydrogel precursor containing a natural component selected from collagen, gelatin, and the like and a second hydrogel precursor containing an electrophilic functional group selected from the group consisting of N-hydroxysuccinimide and the like, where the first hydrogel precursor reacts with the second hydrogel precursor to form the tissue scaffold.

Patent Document 3 discloses a glycosaminoglycan-polycation complex for a tissue regeneration matrix, which is formed by a uniform crosslinking under physiological conditions with a polyfunctional crosslinking agent having two or more electrophilic leaving groups at the carboxyl terminal of polyethylene glycol.

Patent Document 4 discloses a composition (however, a composition using TGF-β is excluded) for cartilage regeneration having fluidity at the time of administration, which uses (a) a monovalent metal salt of low endotoxin alginic acid having an endotoxin content of 100 EU/g or less and (b) SDF-1 in combination.

### Prior Art Documents

### Patent Documents

Patent Document 1: JP2011-245311A
Patent Document 2: JP2011-078764A
Patent Document 3: JP2003-055401A
Patent Document 4: JP6116009B

### SUMMARY OF THE INVENTION

Gelatins described in Patent Documents 1 and 2 and the like generally have a large molecular weight distribution and require a high concentration of a crosslinking agent for gel forming. A crosslinking agent having a high concentration is often toxic to cells and is not preferable as an implantation material for a living body. Another problem is that a crosslinking agent having a high concentration takes time for decomposition in a living body and may be obstructive to tissue regeneration. Hyaluronic acid or an alginate is used in Patent Documents 3 and 4, but hyaluronic acid and the alginate are also materials derived from a living body, and thus there remains the problem that the possibility of the contamination of unknown virus or the residual of endotoxin cannot be completely eliminated.

An object of the present invention is to provide a gel forming kit capable of regenerating hyaline cartilage. Another object of the present invention is to provide a gel that is formed by using the gel forming kit, and a method for producing a gel using the gel forming kit.

As a result of diligent studies to solve the above problems, the inventors of the present invention have found that a gel that is formed by crosslinking a recombinant peptide with a crosslinking agent having at least two chains each of which includes a functional group capable of being covalently bonded to an amino group and includes a hydrophilic linking group exhibits good adhesiveness at the defective part, and this makes it possible to regenerate hyaline cartilage in a minimally invasive manner without using foreign components. The present invention has been completed based on these findings.

That is, according to the present invention, the following inventions are provided.
(1) A gel forming kit comprising: a crosslinking agent having at least two chains each of which includes a functional group capable of being covalently bonded to an amino group and includes a hydrophilic linking group; and a recombinant peptide.
(2) The gel forming kit according to (1), in which the crosslinking agent and the recombinant peptide each have an injectable form.
(3) The gel forming kit according to (1) or (2), in which a combination of the crosslinking agent and the recombinant peptide exhibits a time-dependent gelation ability.
(4) The gel forming kit according to any one of (1) to (3), in which the crosslinking agent has a weight-average molecular weight of 5,000 to 30,000.
(5) The gel forming kit according to any one of (1) to (4), in which the chain which includes a functional group capable of being covalently bonded to an amino group and includes a hydrophilic linking group is represented by Formula 1,

   Z-(A¹)_{w}-(B¹)ₓ-(C¹)_{y}- Formula 1

   in the formula, Z is a functional group capable of being covalently bonded to an amino group, A¹ is a hydrophobic linking group, B¹ is a hydrophilic linking group, C¹ is a hydrophobic linking group, w is an integer of 1 or more, x is an integer of 1 or more, and y is an integer of 0 or more.
(6) The gel forming kit according to any one of (1) to (5), in which the functional group capable of being covalently bonded to an amino group is an isocyanate, an isothiocyanate, a sulfonyl chloride, an aldehyde, an acyl azide, an acid anhydride, an imide ester, an epoxide, or an active ester.
(7) The gel forming kit according to any one of (1) to (6), in which the hydrophilic linking group includes an ethylene oxide unit.
(8) The gel forming kit according to any one of (1) to (7), in which the recombinant peptide is a recombinant gelatin.
(9) The gel forming kit according to (8), in which the recombinant gelatin is represented by the following formula,

   A-[(Gly-X-Y)n]m-B

   in the formula, A represents any amino acid or amino acid sequence, B represents any amino acid or amino acid sequence, n pieces of X's each independently represent any one of amino acids, n pieces of Y's each independently represent any one of amino acids, n represents an integer of 3 to 100, m represents an integer of 2 to 10, and n pieces of Gly-X-Y's may be the same or different from each other.
(10) The gel forming kit according to (8) or (9), in which the recombinant gelatin is any one of the followings:
   a peptide consisting of an amino acid sequence set forth in SEQ ID NO: 1;
   a peptide consisting of an amino acid sequence which is obtained by deleting, substituting, or adding one or several amino acids in the amino acid sequence set forth in SEQ ID NO: 1, and having biocompatibility; or
   a peptide consisting of an amino acid sequence which has 80% or more of a sequence identity with the amino acid sequence set forth in SEQ ID NO: 1, and having biocompatibility.
(11) The gel forming kit according to any one of (1) to (10), in which the gel forming kit is for use as a cartilage restoring agent.
(12) The gel forming kit according to any one of (1) to (11), in which the gel forming kit is for use as a cartilage restoring agent for promoting cartilage regeneration after performing bone marrow stimulation.
(13) The gel forming kit according to any one of (1) to (12), further comprising a cell.
(14) The gel forming kit according to (13), in which the cell is a synovia-derived cell.
(15) The gel forming kit according to (13) or (14), in which a concentration of the cell with respect to a gel at a time of implantation is 1.0 × 10³ to 1.0 × 10⁷ cells / 40 µL.
(16) A gel formed by crosslinking a recombinant peptide with a crosslinking agent having at least two chains each of which includes a functional group capable of being covalently bonded to an amino group and includes a hydrophilic linking group.
(17) The gel according to (16), in which the crosslinking agent has a weight-average molecular weight of 5,000 to 30,000.
(18) The gel according to (16) or (17), in which the chain which includes a functional group capable of being covalently bonded to an amino group and includes a hydrophilic linking group is represented by Formula 1,

   Z-(A¹)_{w}-(B¹)ₓ-(C¹)_{y}- Formula 1

   in the formula, Z is a functional group capable of being covalently bonded to an amino group, A¹ is a hydrophobic linking group, B¹ is a hydrophilic linking group, C¹ is a hydrophobic linking group, w is an integer of 1 or more, x is an integer of 1 or more, and y is an integer of 0 or more.
(19) The gel according to any one of (16) to (18), in which the functional group capable of being covalently bonded to an amino group is an isocyanate, an isothiocyanate, a sulfonyl chloride, an aldehyde, an acyl azide, an acid anhydride, an imide ester, an epoxide, or an active ester.
(20) The gel according to any one of (16) to (19), in which the hydrophilic linking group includes an ethylene oxide unit.
(21) The gel according to any one of (16) to (20), in which the recombinant peptide is a recombinant gelatin.
(22) The gel according to (21), in which the recombinant gelatin is represented by the following formula,

   A-[(Gly-X-Y)n]m-B

   in the formula, A represents any amino acid or amino acid sequence, B represents any amino acid or amino acid sequence, n pieces of X's each independently represent any one of amino acids, n pieces of Y's each independently represent any one of amino acids, n represents an integer of 3 to 100, m represents an integer of 2 to 10, and n pieces of Gly-X-Y's may be the same or different from each other.
(23) The gel according to (21) or (22), in which the recombinant gelatin is any one of the followings:
   a peptide consisting of an amino acid sequence set forth in SEQ ID NO: 1;
   a peptide consisting of an amino acid sequence which is obtained by deleting, substituting, or adding one or several amino acids in the amino acid sequence set forth in SEQ ID NO: 1, and having biocompatibility; or
   a peptide consisting of an amino acid sequence which has 80% or more of a sequence identity with the amino acid sequence set forth in SEQ ID NO: 1, and having biocompatibility.
(24) The gel according to any one of (16) to (23), in which a concentration of the crosslinking agent in the gel is 0.75% by mass to 3.0% by mass.
(25) The gel according to any one of (16) to (24), in which the gel is for use as a cartilage restoring agent.
(26) The gel according to any one of (16) to (25), in which the gel is for use as a cartilage restoring agent for promoting cartilage regeneration after performing bone marrow stimulation.
(27) The gel according to any one of (16) to (26), further comprising a cell.
(28) The gel according to (27), in which the cell is a stem cell or a chondrocyte.
(29) The gel according to (27) or (28), in which the cell is a mesenchymal stem cell.
(30) The gel according to any one of (27) to (29), in which the cell is a synovia-derived cell.
(31) A method for producing the gel according to any one of (16) to (30), the method comprising: mixing a crosslinking agent having at least two chains each of which includes a functional group capable of being covalently bonded to an amino group and includes a hydrophilic linking group with a recombinant peptide and performing a crosslinking reaction.

According to the gel forming kit, the gel, and the method for producing a gel according to aspects of the present invention, hyaline cartilage can be regenerated.

### BRIEF DESCRIPTION OF THE DRAWINGS

Fig. 1 shows an electrophoresis pattern of various substances. The recombinant peptide is CBE3.
Fig. 2 shows a relationship between a concentration of a phosphate buffer solution and a gelation time. Black circles indicate low concentrations, black triangles indicate medium concentrations, and black squares indicate high concentrations.
Fig. 3 shows a result of staining evaluation of a gel brought into contact with a porcine cartilage piece.
Fig. 4 shows an appearance of human bone marrow-derived mesenchymal stem cells seeded on a gel.
Fig. 5 shows results of a live/dead assay of human bone marrow-derived mesenchymal stem cells cultured in various gels. Green: live cells / red: dead cells. Medium concentration CBE3 gel: CBE3 (37.23 mg / mL) and PEG (30 mg / mL), low concentration CBE3 gel: CBE3 (18.62 mg / mL) and PEG (15 mg / mL)
Fig. 6 shows scoring results of cartilage restoration.
Fig. 7 shows a type II collagen (Col II) immunostaining image of cartilage tissue. Brown indicates a Col II positivity.

### DESCRIPTION OF THE PREFERRED EMBODIMENTS

Hereinafter, the embodiments of the present invention will be described in detail.

### [Gel forming kit]

A gel forming kit according to the embodiment of the present invention contains: a crosslinking agent having at least two chains each of which includes a functional group capable of being covalently bonded to an amino group and includes a hydrophilic linking group; and a recombinant peptide.

In the present invention, it has been demonstrated that a gel that is formed by crosslinking a recombinant peptide with a crosslinking agent having at least two chains each of which includes a functional group capable of being covalently bonded to an amino group and includes a hydrophilic linking group exhibits good cartilage restoring effect at the defective part. Since a recombinant peptide is used in the present invention, hyaline cartilage can be regenerated in a minimally invasive manner without using foreign components.

In the gel forming kit according to the embodiment of the present invention, the crosslinking agent and the recombinant peptide each can be contained as an injectable form. "Injectable" means that a substance can pass through a syringe needle, the substance is preferably a fluid solution or a suspension and particularly preferably a homogeneous aqueous solution. Examples of the injectable form include a solution, a suspension, and a powder; however, the injectable form is not particularly limited thereto. In the case of the powder, the powder can be used after being dissolved or suspended in a liquid at the time of use.

The combination of the crosslinking agent and the recombinant peptide may be a combination that exhibits a time-dependent gelation ability. The time-dependent gelation ability means that the gel is gelated at the injection site from immediately after injection to 60 minutes, more preferably between 5 and 10 minutes.

From the viewpoint of gelation at the body temperature of the animal, the crosslinking agent and the recombinant peptide are preferably gelated at 30°C to 50°C, more preferably gelated at 30°C to 40°C, and most preferably gelated at 35°C to 40°C.

### (Crosslinking agent)

The crosslinking agent used in the present invention has at least two chains each of which includes a functional group capable of being covalently bonded to an amino group and includes a hydrophilic linking group. The number of chains (that is, the number of branched structures) which include a functional group capable of being covalently bonded to an amino group and includes a hydrophilic linking group is not particularly limited as long as it is 2 or more and may be 2, 3, 4, 5, 6, 7, 8, 9, 10, or more; however, 2 to 8 is preferable, and 2 to 6 is more preferable. Since a uniform three-dimensional network structure can be formed in a case of using a four-branched polymer, the number is most preferably 4.

In a case of having two or more chains described above, the crosslinking agent has two or more functional groups capable of being covalently bonded to an amino group.

The weight-average molecular weight of the crosslinking agent is not particularly limited; however, it is preferably 5,000 to 40,000, more preferably 5,000 to 30,000, still more preferably 10,000 to 30,000, and particularly preferably 15,000 to 25,000, since a uniform network structure can be formed. One example thereof is 20,000.

The chain which includes a functional group capable of being covalently bonded to an amino group and includes a hydrophilic linking group is preferably represented by Formula 1.

Z-(A¹)_{w}-(B¹)ₓ-(C¹)_{y}- Formula 1

in the formula, Z is a functional group capable of being covalently bonded to an amino group, A¹ is a hydrophobic linking group, B¹ is a hydrophilic linking group, C¹ is a hydrophobic linking group, w is an integer of 1 or more, x is an integer of 1 or more, and y is an integer of 0 or more.

The crosslinking agent is preferably represented by Formula 2.

[Z-(A¹)_{w}-(B¹)ₓ-(C¹)_{y}-]ᵥ-CHₙ Formula 2

In the formula, Z is a functional group capable of being covalently bonded to an amino group, A¹ is a hydrophobic linking group, B¹ is a hydrophilic linking group, C¹ is a hydrophobic linking group, w is an integer of 1 or more, x is an integer of 1 or more, y is an integer of 0 or more, v is an integer of 2 to 4, and n is an integer of 0 to 2. However, v + n is 4.

Z, A¹, B¹, and C¹ may be the same or different in each branch or between branches, and w, x, and y may be the same or different between branches.

w is preferably an integer of 1 to 10 and more preferably an integer of 1 to 5.

x is preferably an integer of 10 to 300 and more preferably an integer of 20 to 200.

y is preferably an integer of 0 to 5 and more preferably an integer of 0 to 3.

v is preferably 4, and n is preferably 0.

Examples of the functional group capable of being covalently bonded to an amino group include an isocyanate, an isothiocyanate, a sulfonyl chloride, an aldehyde, an acyl azide, an acid anhydride, an imide ester, an epoxide, and an active ester; however, the functional group is not particularly limited thereto. From the viewpoint that the reaction easily proceeds at a pH of a living body, the functional group capable of being covalently bonded to an amino group is more preferably a succinimidyl group. The crosslinking agent has two or more functional groups capable of being covalently bonded to an amino group, and the functional groups may be the same or different.

Examples of the hydrophilic linking group include an ethylene oxide group (-CH₂CH₂O-) and a group including an ethylene oxide unit; however, the hydrophilic linking group is not particularly limited thereto. The crosslinking agent in a case where the hydrophilic linking group is an ethylene oxide group (-CH₂CH₂O-) or a group including an ethylene oxide unit is also referred to as a polyethylene glycol (PEG) crosslinking agent. The crosslinking agent is preferably a PEG crosslinking agent. In a case where a PEG crosslinking agent is used, a crosslinking agent other than the PEG crosslinking agent may be used in combination.

Examples of the hydrophobic linking group represented by A¹ include a hydrocarbon group having 1 to 3 carbon atoms, and a methylene group or an ethylene group is preferable. The hydrophobic linking group represented by A¹ may have a linking group, for example, -O-, -CO-, or -COO- at the terminal.

Examples of the hydrophobic linking group represented by C¹ include a hydrocarbon group having 1 to 3 carbon atoms, and a methylene group or an ethylene group is preferable. The hydrophobic linking group represented by C¹ may have a linking group, for example, -O-, -CO-, or -COO- at the terminal.

The crosslinking agent used in the present invention preferably has tissue adhesiveness. The tissue adhesiveness means that the crosslinking agent can be chemically or physically bonded to the tissue at the installation site during installation. Preferably, it is a chemical bonding between the crosslinking agent and the amino group of the tissue surface protein.

### (Recombinant peptide)

The recombinant peptide used in the present invention is preferably a recombinant peptide having biocompatibility. The biocompatibility means that when the recombinant peptide is brought into contact with a living body, a noticeable adverse reaction such as a long-term and chronic inflammatory reaction is not elicited.

The recombinant peptide may be devised to enhance cell adhesiveness. Specifically, a method such as "coating of the base surface with a cell adhesion substrate (fibronectin, vitronectin, laminin) or a cell adhesion sequence (an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence, which are represented by one-letter notation of amino acids) peptide", "amination and cationization of the base surface", or "plasma treatment of the base surface and hydrophilic treatment by corona discharge" can be used.

The recombinant peptide is preferably a peptide containing lysine and more preferably a peptide containing 5% or more of lysine, in order for a functional group capable of being covalently bonded to an amino group (a succinimidyl group or the like) to be capable of being subjected to a reaction.

The kind of recombinant peptide is not particularly limited; however, for example, gelatin, collagen, elastin, fibronectin, ProNectin, laminin, tenascin, fibrin, fibroin, entactin, thrombospondin, and RetroNectin are preferable, gelatin, collagen, and atelocollagen are more preferable, and gelatin is most preferable. Recombinant gelatin will be described later in the present specification.

The hydrophilicity value "1/IOB" of the recombinant peptide used in the present invention is preferably 0 to 1.0. The hydrophilicity value is more preferably 0 to 0.6 and still more preferably 0 to 0.4. IOB is an index of hydrophilicity/hydrophobicity based on the organic conceptual diagram illustrating the polarity/non-polarity of an organic compound, which has been proposed by Minoru Fujita, and the details thereof are described in, for example, "Pharmaceutical Bulletin", vol. 2, 2, pp. 163-173 (1954), "Journal of Japanese Chemistry" vol. 11, 10, pp. 719-725 (1957), and "Fragrance Journal", vol. 50, pp. 79-82 (1981). A brief description of IOB is as follows. Assuming that the source of all organic compounds is methane (CH₄), all other compounds are regarded as derivatives of methane, and predetermined values are respectively set for the number of carbon atoms, the substituent, transformation moiety, and the ring of the compounds. Then, the scores are added to obtain the organic value (OV) and the inorganic value (IV), and these values are plotted on a diagram in which the organic value is in the X axis and the inorganic value is in the Y axis. The IOB in the organic conceptual diagram means the ratio of the inorganic value (IV) to the organic value (OV) in the organic conceptual diagram, that is, "the inorganic value (IV) / the organic value (OV)". For details on the organic conceptual diagram, "Organic conceptual diagram -basics and applications-, New edition" (written by Yoshio Koda et al., Sankyo Publishing Co., Ltd., 2008) is referred to. In the present specification, hydrophilicity/hydrophobicity is represented by the reciprocal of IOB, the "1/IOB" value. The smaller the "1/IOB" value (closer to 0) is, the more hydrophilic it is.

In a case where the "1/IOB" value of the recombinant peptide used in the present invention is adjusted in the above range, hydrophilicity is high and water absorbency is high.

The recombinant peptide used in the present invention is preferably 0.3 or less and -9.0 or more and more preferably 0.0 or less and -7.0 or more, in terms of the hydrophilicity/hydrophobicity index represented by Grand average of hydropathicity (GRAVY) value. The Grand average of hydropathicity (GRAVY) value can be obtained by a method described in "Gasteiger E., Hoogland C., Gattiker A., Duvaud S., Wilkins M. R., Appel R. D., Bairoch A.; Protein Identification and Analysis Tools on the ExPASy Server; (In) John M. Walker (ed): The Proteomics Protocols Handbook, Humana Press (2005). pp. 571-607" or "Gasteiger E., Gattiker A., Hoogland C., Ivanyi I., Appel R. D., Bairoch A.; ExPASy: the proteomics server for in-depth protein knowledge and analysis.; Nucleic Acids Res. 31: 3784-3788 (2003)".

In a case where the GRAVY value of the recombinant peptide used in the present invention is adjusted in the above range, hydrophilicity is high and water absorbency is high.

### (Recombinant gelatin)

The recombinant peptide is preferably recombinant gelatin.

The recombinant gelatin means a polypeptide or a protein-like substance, which has a gelatin-like amino acid sequence prepared by gene recombination technique. The recombinant gelatin that can be used in the present invention preferably has a repeat of the sequence represented by Gly-X-Y (X and Y each independently represent any one of amino acids), which is characteristic of collagen. Here, a plurality of Gly-X-Y's may be the same or different from each other. Preferably, two or more sequences of cell adhesion signals are contained in one molecule. As the recombinant gelatin used in the present invention, gelatin having an amino acid sequence derived from the partial amino acid sequence of collagen can be used. For example, recombinant gelatins disclosed in EP1014176A2, US6992172B, WO2004/085473A, WO2008/103041A, and the like can be used but are not limited thereto. The recombinant gelatin used in the present invention is preferably gelatin having the following aspects.

Recombinant gelatin has excellent biocompatibility due to the original performance of natural gelatin and is excellent in non-infectivity due to not being natural gelatin since there is no concern about bovine spongiform encephalopathy (BSE) or the like. Further, since recombinant gelatin is more homogeneous than natural gelatin and the sequence is determined, it is possible to precisely design recombinant gelatin having a more uniform strength and decomposability by crosslinking or the like.

The molecular weight of the recombinant gelatin is not particularly limited; however it is preferably 2,000 or more and 100,000 or less (2 kDa or more and 100 kDa or less), more preferably 2,500 or more and 95,000 or less (2.5 kDa or more and 95 kDa or less), still more preferably 5,000 or more and 90,000 or less (5 kDa or more and 90 kDa or less), and most preferably 10,000 or more and 90,000 or less (10 kDa or more and 90 kDa or less).

The molecular weight distribution of the recombinant gelatin is not particularly limited; however, it is preferable that in the molecular weight distribution measurement, recombinant gelatin has an area proportion of the maximum molecular weight peak of 70% or more, more preferably 90% or more, and most preferably 95% or more, with respect to the total area of all the molecular weight peaks. The molecular weight distribution of recombinant gelatin can be measured by the method disclosed in WO2017/012284A.

Recombinant gelatin preferably has a repeat of the sequence represented by Gly-X-Y, which is characteristic of collagen. Here, a plurality of Gly-X-Y's may be the same or different from each other. In Gly-X-Y, Gly indicates glycine, and X and Y indicate any amino acid (preferably any amino acid other than glycine). The sequence represented by Gly-X-Y, which is characteristic of collagen, is a partial structure very specific in the composition and sequence of the amino acids of gelatin and collagen as compared with other proteins. In this part, glycine occupies about one-third of the total and is repeated in every three amino acids in the amino acid sequence. Glycine is the simplest amino acid and has few restrictions in the arrangement of molecular chains, which greatly contributes to the regeneration of the helix structure during gelation. Amino acids represented by X and Y include a large quantity of imino acids (proline and oxyproline) and preferably occupy 10% to 45% of the total. The amino acids of the repeating structure of Gly-X-Y occupy preferably 80% or more, more preferably 95% or more, and most preferably 99% or more in the sequence of the recombinant gelatin.

In general gelatin, charged amino acids and uncharged amino acids among the polar amino acids are present at a ratio of 1:1. Here, the polar amino acids specifically refer to cysteine, aspartic acid, glutamic acid, histidine, lysine, asparagine, glutamine, serine, threonine, tyrosine, and arginine. Among them, the polar uncharged amino acids refer to cysteine, asparagine, glutamine, serine, threonine, and tyrosine. In the gelatin used in the present invention, the proportion of polar amino acids to all the constituent amino acids is 10% to 40% and preferably 20% to 30%. In addition, the proportion of uncharged amino acids in the polar amino acids is 5% or more and less than 20% and preferably 5% or more and less than 10%. Further, the sequence preferably does not contain therein any one amino acid of serine, threonine, asparagine, tyrosine, or cysteine and more preferably two or more thereof.

In general, the minimum amino acid sequence that acts as a cell adhesion signal in a polypeptide is known (for example, "Pathophysiology" Vol. 9, No. 7 (1990), page 527) published by Nagai Shoten Co., Ltd.). The gelatin used in the present invention preferably has two or more of these cell adhesion signals in one molecule. The specific sequence of the cell adhesion signal is preferably a sequence, for example, an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence, which are represented by one-letter notation of amino acids, since there are many kinds of cells which adhere to these sequences. An RGD sequence, a YIGSR sequence, a PDSGR sequence, an LGTIPG sequence, an IKVAV sequence, and an HAV sequence are more preferable, and an RGD sequence is particularly preferable. Among the RGD sequences, an ERGD sequence is preferable. In a case where gelatin having a cell adhesion signal is used, the amount of substrate produced by cells can be increased. For example, in a case of cartilage differentiation using a mesenchymal stem cell as a cell, the production of glycosaminoglycan (GAG) can be increased.

Regarding the arrangement of RGD sequences in the recombinant gelatin used in the present invention, it is preferable that the number of amino acids between RGDs is not uniform between 0 and 100 and preferably between 25 and 60.

From the viewpoint of cell adhesion and proliferation, the content of the above minimum amino acid sequence is preferably 3 to 50 sequences, more preferably 4 to 30 sequences, and particularly preferably 5 to 20 sequences in one protein molecule. 12 sequences are most preferable.

In the recombinant gelatin used in the present invention, the proportion of RGD motif to the total number of amino acids is preferably at least 0.4%. In a case where the recombinant gelatin contains 350 or more amino acids, each stretch of 350 amino acids preferably contains at least one RGD motif. The proportion of RGD motif to the total number of amino acids is more preferably at least 0.6%, still more preferably at least 0.8%, still more preferably at least 1.0%, still more preferably at least 1.2%, and most preferably at least 1.5%. The number of RGD motifs in the peptide is preferably at least 4, more preferably at least 6, still more preferably at least 8, and even still more preferably 12 or more and 16 or less per 250 amino acids. A proportion of 0.4% of the RGD motif corresponds to at least one RGD sequence per 250 amino acids. Since the number of RGD motifs is an integer, the recombinant gelatin consisting of 251 amino acids have to contain at least two RGD sequences to satisfy the feature of at least 0.4%. The recombinant gelatin preferably contains at least 2 RGD sequences per 250 amino acids, more preferably contains at least 3 RGD sequences per 250 amino acids, and still more preferably at least 4 RGD sequences per 250 amino acids. Further, another aspect of the recombinant gelatin in the present invention includes at least 4 RGD motifs, preferably at least 6 RGD motifs, more preferably at least 8 RGD motifs, and still more preferably 12 or more and 16 or less RGD motifs.

### Recombinant gelatin may be partially hydrolyzed.

The recombinant gelatin used in the present invention is preferably represented by A-[(Gly-X-Y)ₙ]ₘ-B. The n pieces of X's each independently represent any one of amino acids, and the n pieces of Y's each independently represent any one of the amino acids. m preferably represents an integer of 2 to 10 and more preferably an integer of 3 to 5. n is preferably an integer of 3 to 100, more preferably an integer of 15 to 70, and most preferably an integer of 50 to 65. A represents any amino acid or amino acid sequence, and B represents any amino acid or amino acid sequence. n pieces of Gly-X-Y's may be the same or different from each other.

More preferably, the recombinant gelatin used in the present invention is recombinant gelatin represented by Formula: Gly-Ala-Pro-[(Gly-X-Y)₆₃]₃-Gly (in the formula, 63 X's each independently represent any one of amino acids, 63 Y's each independently represent any one of amino acids. 63 Gly-X-Y's may be the same or different from each other).

A plurality of naturally occurring collagen sequence units are preferably bound to the repeating unit. The naturally occurring collagen referred to here may be any naturally occurring collagen as long as it is naturally present; however, it is preferably type I, type II, type III, type IV, or type V collagen. It is more preferably type I, type II, or type III collagen. According to another aspect, the origin of the collagen described above is preferably human, bovine, porcine, mouse, or rat and more preferably human.

The isoelectric point of the recombinant gelatin used in the present invention is preferably 5 to 10, more preferably 6 to 10, and still more preferably 7 to 9.5. The measurement of the isoelectric point of recombinant gelatin can be performed by passing a solution of 1% by mass of recombinant gelatin through a mixed crystal column of a cation and anion exchange resins and then measuring the pH according to isoelectric focusing (see Maxey, C. R. (1976); Phitogr. Gelatin 2, Editor Cox, P. J. Academic, London, Engl.).

The recombinant gelatin is preferably not deaminated.

The recombinant gelatin preferably has no telopeptide.

The recombinant gelatin is preferably a substantially pure polypeptide prepared from a nucleic acid encoding an amino acid sequence.

The recombinant gelatin used in the present invention is particularly preferably any one of the followings:
(1) a peptide consisting of an amino acid sequence set forth in SEQ ID NO: 1;
(2) a peptide consisting of an amino acid sequence which is obtained by deleting, substituting, or adding one or several amino acids in the amino acid sequence set forth in SEQ ID NO: 1, and having biocompatibility; or
(3) a peptide consisting of an amino acid sequence which has 80% or more (more preferably 90% or more, particularly preferably 95% or more, and most preferably 98% or more) of a sequence identity with the amino acid sequence set forth in SEQ ID NO: 1, and having biocompatibility.

The "one or several" in the "amino acid sequence which is obtained by deleting, substituting, or adding one or several amino acids" is preferably 1 to 20, more preferably 1 to 10, still more preferably 1 to 5, and particularly preferably 1 to 3.

The recombinant gelatin used in the present invention can be produced by a gene recombination technique known to those skilled in the art, and it can be produced, for example, according to the method disclosed in EP1014176A2, US6992172B, WO2004/085473A, WO2008/103041A, and the like. Specifically, a gene encoding an amino acid sequence of a predetermined recombinant gelatin is obtained, the gene is incorporated into an expression vector to prepare a recombinant expression vector, and the expression vector is introduced into a proper host to prepare a transformant. Recombinant gelatin is produced by culturing the obtained transformant in a suitable medium, and the gelatin used in the present invention can be prepared by recovering the recombinant gelatin produced, from the culture.

### (Cartilage restoring agent)

The gel forming kit according to the embodiment of the present invention can be used as a cartilage restoring agent and particularly as a cartilage restoring agent for promoting cartilage regeneration after performing bone marrow stimulation.

The gel forming kit according to the embodiment of the present invention can be used for various applications as long as the cartilage formation promoting effect can be effectively utilized.

Cartilage is present in tubular structures such as a joint, a thoracic wall, an intervertebral disc, a meniscus, larynx, an airway, and ears and can be classified into three types; hyaline cartilage, elastic cartilage, and fibrous cartilage. For example, articular cartilage is hyaline cartilage and consists of a chondrocyte, a collagenous extracellular matrix, a proteoglycan, and water, with no blood vessels being distributed. Hyaline cartilage is rich in type II collagen and has characteristics such as being stained with an anti-type II collagen antibody and being stained red with a safranin-O staining that stains proteoglycan.

The gel forming kit according to the embodiment of the present invention is useful for treating such as cartilage diseases associated with cartilage dysfunction due to degeneration or destruction of cartilage, damage and/or resection of cartilage due to trauma or surgery, and congenital hypoplasia or malformation of cartilage. Further, in cartilage defect restoration by cell implantation, the kit is useful for inducing cartilage from an undifferentiated progenitor cell or maintaining the character of the chondrocyte. Examples of such cartilage diseases include osteoarthritis, chronic rheumatoid arthritis, osteochondritis dissecans, damage to articular cartilage due to trauma, and herniated disc. Examples of the congenital hypoplasia and malformation of cartilage include anotia and microtia. The gel forming kit according to the embodiment of the present invention can be used for treatment or as a prophylactic agent for a cartilage-associated disease.

In a case of being used as a cartilage restoring agent, a crosslinking agent and a recombinant peptide of the gel forming kit according to the embodiment of the present invention are mixed and then can be locally administered to a subject.

The gel forming kit according to the embodiment of the present invention may further contain an instruction for administering a crosslinking agent having at least two chains each of which includes a functional group capable of being covalently bonded to an amino group and includes a hydrophilic linking group and a recombinant peptide to a subject.

The present invention provides a method for restoring cartilage, including administering a mixture of a crosslinking agent having at least two chains each of which includes a functional group capable of being covalently bonded to an amino group and includes a hydrophilic linking group and a recombinant peptide to a subject in need of restoration of cartilage.

The present invention provides a method for restoring cartilage, including simultaneously or sequentially administering a crosslinking agent having at least two chains each of which includes a functional group capable of being covalently bonded to an amino group and includes a hydrophilic linking group and a recombinant peptide to a subject in need of restoration of cartilage.

The present invention provides a method for treating or preventing a cartilage-associated disease, including administering a mixture of a crosslinking agent having at least two chains each of which includes a functional group capable of being covalently bonded to an amino group and includes a hydrophilic linking group and a recombinant peptide to a subject.

The present invention provides a method for treating or preventing a cartilage-associated disease, including simultaneously or sequentially administering a crosslinking agent having at least two chains each of which includes a functional group capable of being covalently bonded to an amino group and includes a hydrophilic linking group and a recombinant peptide to a subject.

The present invention provides a gel forming kit containing a crosslinking agent having at least two chains each of which includes a functional group capable of being covalently bonded to an amino group and includes a hydrophilic linking group and a recombinant peptide, which is for use for the restoration of cartilage.

The present invention provides a gel forming kit containing a crosslinking agent having at least two chains each of which includes a functional group capable of being covalently bonded to an amino group and includes a hydrophilic linking group and a recombinant peptide, which is for use for treating or preventing a cartilage-associated disease.

The present invention provides a gel formed by crosslinking of a recombinant peptide with a crosslinking agent having at least two chains each of which includes a functional group capable of being covalently bonded to an amino group and includes a hydrophilic linking group, which is for use for the restoration of cartilage.

The present invention provides a gel formed by crosslinking of a recombinant peptide with a crosslinking agent having at least two chains each of which includes a functional group capable of being covalently bonded to an amino group and includes a hydrophilic linking group, which is for use for treating or preventing a cartilage-associated disease.

The present invention provides a usage of a gel forming kit including a crosslinking agent having at least two chains each of which includes a functional group capable of being covalently bonded to an amino group and includes a hydrophilic linking group and a recombinant peptide, which is for producing a cartilage restoring agent.

The present invention provides a usage of a gel forming kit including a crosslinking agent having at least two chains each of which includes a functional group capable of being covalently bonded to an amino group and includes a hydrophilic linking group and a recombinant peptide, which is for treating a cartilage-associated disease or producing a prophylactic agent for a cartilage-associated disease.

The present invention provides a usage of a gel formed by crosslinking of a recombinant peptide with a crosslinking agent having at least two chains each of which includes a functional group capable of being covalently bonded to an amino group and includes a hydrophilic linking group, which is for producing a cartilage restoring agent.

The present invention provides a usage of a gel formed by crosslinking of a recombinant peptide with a crosslinking agent having at least two chains each of which includes a functional group capable of being covalently bonded to an amino group and includes a hydrophilic linking group, which is for treating a cartilage-associated disease or producing a prophylactic agent for a cartilage-associated disease.

### <Gel and method for producing gel>

The present invention provides a gel formed by crosslinking a recombinant peptide with a crosslinking agent having at least two chains each of which includes a functional group capable of being covalently bonded to an amino group and includes a hydrophilic linking group.

Details and preferred aspects of the crosslinking agent and the recombinant peptide are as described above in the present specification.

The gel according to the embodiment of the present invention can be preferably used as a cartilage restoring agent and more preferably as a cartilage restoring agent for promoting cartilage regeneration after performing bone marrow stimulation. Details of the cartilage restoring agent are as described above in the present specification.

The concentration (final concentration) of the crosslinking agent in the gel according to the embodiment of the present invention is preferably 0.75% by mass to 6% by mass, more preferably 0.75% by mass to 3.0% by mass, and still more preferably 0.75% by mass to 1.5% by mass. In addition, the mass concentration of the crosslinking agent with respect to the mass concentration of the recombinant peptide is preferably 0.8 times to 6.5 times, more preferably 0.8 times to 1.6 times, and still more preferably 0.8 times from the viewpoint of cytotoxicity.

The gel according to the embodiment of the present invention can be produced by mixing a crosslinking agent having at least two chains each of which includes a functional group capable of being covalently bonded to an amino group and includes a hydrophilic linking group with a recombinant peptide and performing a crosslinking reaction.

The concentration of the crosslinking agent in the crosslinking reaction solution is preferably in the range of 15 to 60 g/L, more preferably 20 to 50 g/L, still more preferably 25 to 40 g/L, and particularly preferably 25 to 35 g/L. One example thereof is 30 g/L. The active terminal molar concentration ratio [amino group (-NH₂)]: [functional group capable of being covalently bonded to amino group] is in the range of 1:2 to 4:1 and preferably 4:1.

The concentration of the recombinant peptide in the crosslinking reaction solution is preferably 1.9% by mass to 15% by mass, more preferably 1.9% by mass to 7.4% by mass, and still more preferably 1.9% by mass to 3.7% by mass.

The gel may contain a solvent. The solvent is preferably an aqueous solvent, more preferably an aqueous buffer solution, and most preferably a phosphate buffer solution.

The gel according to the embodiment of the present invention may contain cells.

As the cell, any cell can be used as long as the cell can be implanted, and the kind thereof is not particularly limited. In addition, a cell to be used may be one kind, or a plurality of kinds of cells may be used in combination. Further, the cell to be used is preferably an animal cell, more preferably a vertebrate-derived cell, and particularly preferably a human-derived cell. The kind of vertebrate-derived cell (particularly human-derived cell) may be any one of a stem cell, a progenitor cell, or a mature cell, and a stem cell is most preferable. A cell to be implanted is preferably a stem cell, more preferably a mesenchymal stem cell, and most preferably a synovia-derived stem cell.

Examples of the stem cell capable of being used include an embryonic stem (ES) cell, a germline stem (GS) cell, an induced pluripotent stem (iPS) cell, a mesenchymal stem cell (MSC), a hematopoietic stem cell, an amnion cell, an umbilical cord blood cell, and a bone marrow-derived cell, a myocardial stem cell, an adipose-derived stem cell, a synovia-derived stem cell, and a neural stem cells.

Examples of the progenitor cell and mature cell capable of being used include cells derived from skin, dermis, epidermis, muscle, myocardium, nerve, bone, cartilage, endothelium, brain, epithelium, heart, kidney, liver, pancreas, spleen, oral cavity, cornea, bone marrow, umbilical cord blood, amnion, hair, and the like.

Examples of the human-derived cell capable of being used include an ES cell, an iPS cell, MSC, a chondrocyte, an osteoblast, an osteoblast progenitor cell, a mesenchyme cell, a myoblast, a myocardial cell, a myocardial blast cell, a nerve cell, a hepatocyte, a beta cell, a fibroblast, a corneal endothelial cell, a vascular endothelial cell, a corneal epithelial cell, amnion cell, an umbilical cord blood cell, a bone marrow-derived cell, a synovia-derived cell, and a hematopoietic stem cell. Further, the origin of the cell may be either an autologous cell or an allogeneic cell.

In the present invention, a vascular cell can be also used. In the present specification, the vascular cell means a cell involved in angiogenesis and is a cell constituting a blood vessel and blood, or a progenitor cell a somatic stem cell capable of differentiating into this cell. Here, the vascular cell does not include cells that do not naturally differentiate into cells constituting a blood vessel and blood, for example, pluripotent cells such as an ES cell, a GS cell, or an iPS cell, and a mesenchymal stem cell (MSC). The vascular cell is preferably a cell that constitutes a blood vessel. Among the vertebrate-derived cells (particularly human-derived cells), specific examples of the cell constituting a blood vessel include a vascular endothelial cell and a vascular smooth muscle cell. The vascular endothelial cell may be any one of a venous endothelial cell or an arterial endothelial cell. As the progenitor cell of the vascular endothelial cell, a vascular endothelial progenitor cell can be used. The vascular cell is preferably a vascular endothelial cell and a vascular endothelial progenitor cell. As the cell constituting blood, a blood cell can be used, and a white blood cell, for example, a lymphocyte or a neutrophil, a monocyte cell, and a hematopoietic stem cell which is a stem cell thereof can be used.

In the present specification, the non-vascular cell means a cell other than the above-described vascular cell. Examples thereof capable of being used include an ES cell, an iPS cell, a mesenchymal stem cell (MSC), a myocardial stem cell, a myocardial cell, a fibroblast, a myoblast, a chondrocyte, a synovia-derived cell, a hepatocyte, or a nerve cell.

As the cell in the present invention, a stem cell, a chondrocyte, or a synovia-derived cell can be preferably used.

The concentration of cells with respect to the gel at the time of implantation is preferably 1.0 × 10³ to 1.0 × 10⁷ cells / 40 µL, more preferably 1.0 ×10⁴ to 1.0 × 10⁶ cells / 40 µL, most preferably 1.0 × 10⁵ to 5.0 × 10⁵ cells / 40 µL.

The number of cells to be implanted is not limited as long as cells are contained; however it is preferably 1.0 × 10³ to 1.0 × 10⁷ cells, more preferably 1.0 × 10⁴ to 1.0 × 10⁶ cells, and most preferably 1.0 × 10⁵ to 5.0 × 10⁵ cells per defect (diameter 5 mm × depth 2 mm).

The gel according to the embodiment of the present invention may contain a cytokine or may not contain a cytokine.

The kind of cytokine is not particularly limited; however, examples thereof include a hematopoietic factor (for example, Colony-Stimulating Factor (CSF), Granulocyte Colony Stimulating Factor (G-CSF), and Erythropoietin (EPO)); a cell growth factor (for example, Epidermal Growth Factor (EGF), Fibroblast Growth Factor (FGF), Platelet-Derived Growth Factor (PDGF), Hepatocyte Growth Factor (HGF), and Transforming Growth Factor (TGF)); and a neurotrophic factor (for example, Nerve Growth Factor (NGF)).

The gel according to the embodiment of the present invention preferably has a modulus of elasticity of 1 kPa or more, more preferably 3 kPa or more, and most preferably 5 kPa or more from the viewpoint of strength.

The gel according to the embodiment of the present invention preferably has a modulus of elasticity of 15 kPa or less, more preferably 10 kPa or less, and most preferably 6 kPa or less from the viewpoint of not damaging the surrounding tissue.

The gel according to the embodiment of the present invention preferably has a compressive rupture strength of 0.01 MPa or more, more preferably 0.1 MPa or more, and most preferably 0.2 MPa or more from the viewpoint of strength.

Examples of the gel according to the embodiment of the present invention include a gel containing no Stromal cell-derived factor 1 (SDF-1).

The buffer solution for adjusting the gel may be any buffer solution and is preferably a buffer solution which is capable of being shielding amino groups and most preferably a phosphate buffer solution.

The concentration of the buffer solution is not limited; however, it is preferably 0 to 500 mmol/L, more preferably 10 to 300 mmol/L, and most preferably 25 to 75 mmol/L in terms of controlling the gelation time.

The present invention will be further specifically described with reference to Examples, but the present invention is not limited by Examples.

### Examples

### [Reference Example 1] Recombinant peptide (recombinant gelatin)

The following CBE3 was prepared as a recombinant peptide (recombinant gelatin) (disclosed in WO2008/103041A).
CBE3:
Molecular weight: 51.6kD
Structure: GAP[(GXY)₆₃]₃G
Number of amino acids: 571 amino acids
RGD sequence: 12 sequences
Imino acid content: 33%

Almost 100% of amino acids have a repeating structure of GXY. The amino acid sequence of CBE3 does not include serine, threonine, asparagine, tyrosine, and cysteine. CBE3 has an ERGD sequence.
Isoelectric point: 9.34
GRAVY value: -0.682
1/IOB value: 0.323

Amino acid sequence (SEQ ID NO: 1 in the sequence listing) (the same as SEQ ID NO: 3 in WO2008/103041A, however, the X at the terminal is corrected to "P")

GAP(GAPGLQGAPGLQGMPGERGAAGLPGPKGERGDAGPKGADGAPGAPGL
QGMPGERGAAGLPGPKGERGDAGPKGADGAPGKDGVRGLAGPIGPPGERGAAGLPG
PKGERGDAGPKGADGAPGKDGVRGLAGPIGPPGPAGAPGAPGLQGMPGERGAAGLP
GPKGERGDAGPKGADGAPGKDGVRGLAGPP)3G

### [Example 1]

### 1. Preparation of gel

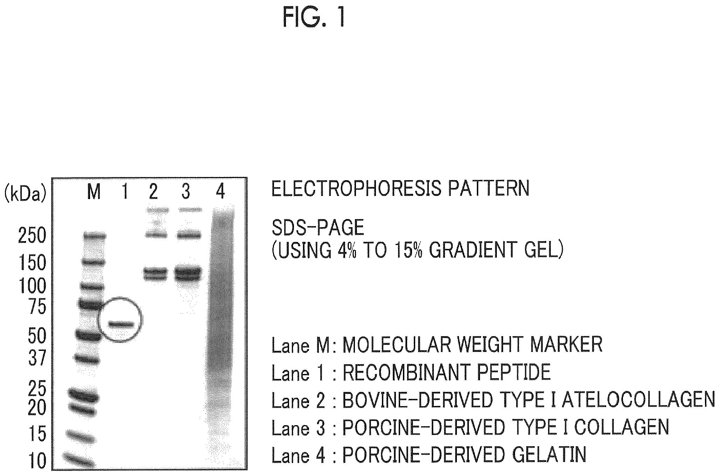

CBE3 (mass average molecular weight (M_{w}) = 51.2 kg/mol, 18.62 to 148.92 g/L, [-NH₂] 12 to 96 mmol/L) and a commercially available PEG crosslinking agent (M_{w} = 20 kg/mol, 120 g/L, [-OSu] 24 mmol/L, trade name: PTE-200HS, NOF CORPORATION) each were dissolved in the phosphate buffered saline (trade name: PBS pH 7.4) (manufactured by Thermo Fisher Scientific) in separate containers. Su represents a succinimidyl group. Solutions having a plurality of concentrations of CBE3 were prepared in advance so that the ratio of the amino group at the terminal of the constituent polymer to the succinimidyl group was 1:2 to 4:1 at the time of dissolution. The molecular weight of CBE3 is about 51 kDa, and as can be seen from the electrophoresis pattern of various substances shown in Fig. 1, CBE3 has a more uniform molecular weight distribution than the porcine-derived gelatin.

The chemical structure (reproduced from the manufacturer's website) of the PEG crosslinking agent used (PTE-200HS, NOF CORPORATION) is shown below. M_{w} is 20,000, and n in the formula is about 114.

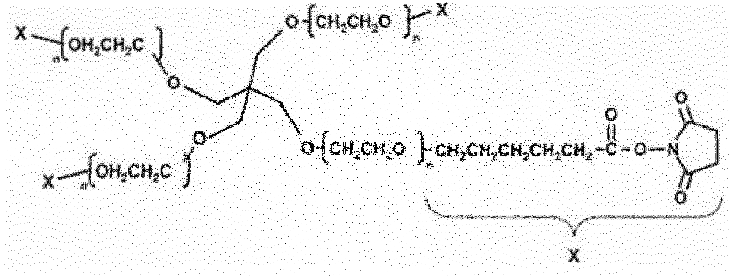

The equal volumes of the CBE3 solution and the PEG crosslinking agent solution obtained above were mixed, and a chemical reaction between CBE3 and the PEG crosslinking agent was carried out to form a three-dimensional network. A schematic diagram of the reaction is shown below.

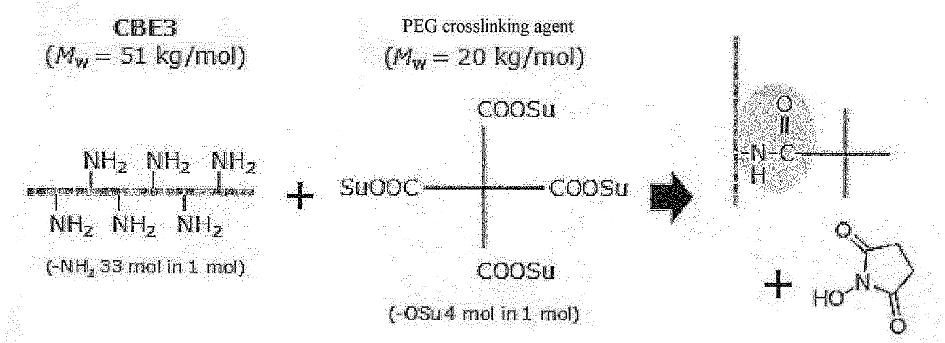

In all cases, a transparent gel (hydrogel) was formed. The vial containing the gelation precursor solution was declined, and the time when the fluidity disappeared was defined as the gelation time. The relationship between the active terminal molar concentration ratio and the gelation time is shown in the table below. The gelation time changed depending on the concentration condition of each precursor. The concentration condition ([-NH₂]:[-OSu] = 4:1) that gave the best reactivity was applied to the subsequent experiments.

**[Table 1]**

| Relationship between active terminal molar concentration ratio and gelation time | | | |
|---|---|---|---|
| PEG crosslinking agent concentration [mg/mL] | CBE3 concentration [mg/mL] | Active terminal molar concentration ratio [-NH₂]:[-OSu] | Gelation time |
| 120 | 18.6 | 1:2 | 12 minutes |
| 120 | 37.2 | 1:1 | 3 minutes |
| 120 | 74.5 | 2:1 | Several seconds |
| 120 | 149 | 4:1 | Immediate |

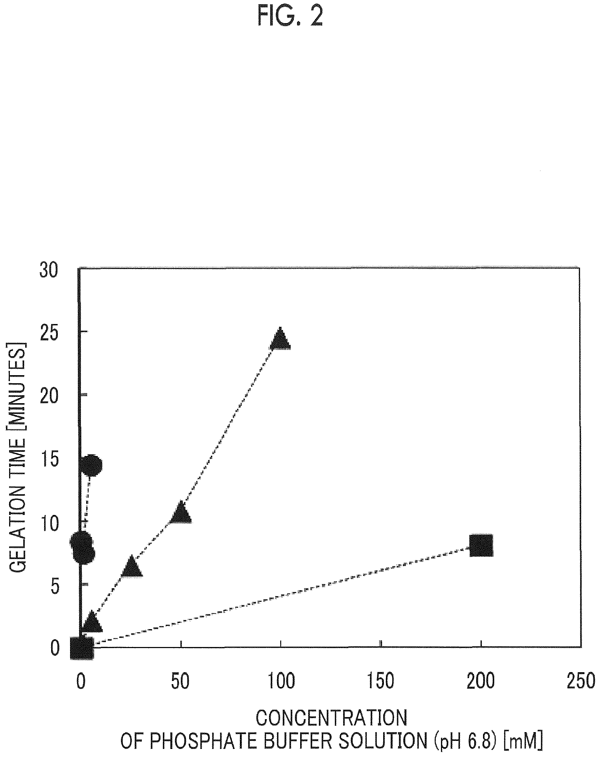

A plurality of aqueous solutions having different solute concentrations were prepared (low/medium/high concentration), and equal volumes of components in each concentration group were mixed at room temperature. Table 2 shows the concentrations of the precursor aqueous solution and the buffer solution used in the gel preparation. In all cases, a gel could be obtained. The gelation time could be controlled by adjusting the buffer solution concentration (Fig. 2). It is presumed that the higher the concentration of the buffer solution is, the more the amino group which is a crosslinking reaction group is shut out, thereby resulting in a delay in the crosslinking reaction. A low concentration gel, a medium concentration gel, and a high concentration gel were respectively obtained by the following solutions being left overnight; a low concentration gelation solution having a combination of a low concentration CBE aqueous solution (18.6 mg/mL) and a PEG crosslinking agent aqueous solution (15 g/L) in a 50 mmol/L phosphate buffer solution; a medium concentration gelation solution having a combination of a medium concentration CBE aqueous solution (37.23 g/L) and a PEG crosslinking agent aqueous solution (30 g/L) in a 50 mmol/L phosphate buffer solution; and a high concentration gelation solution having a combination of a high concentration CBE aqueous solution (74.46 g/L) and a PEG crosslinking agent aqueous solution (60 g/L) in a 50 mmol/L phosphate buffer solution.

**[Table 2]**

| Precursor aqueous solution used for gel preparation and buffer solution concentration | | | |
|---|---|---|---|
| Sample code | CBE3 aqueous solution [g/L] | PEG crosslinking agent aqueous solution [g/L] | Phosphate buffer solution concentration [mmol/L] |
| Low concentration | 18.62 | 15 | 0 to 50 |
| Medium concentration | 37.23 | 30 | 0 to 200 |
| High concentration | 74.46 | 60 | 0 to 200 |

For comparison with existing materials, when the experiment was carried out by changing the crosslinking target from CBE3 to medical gelatin (trade name: High Grade gelatin APAT, Nippi. Inc., medium molecular weight M_{w} = about 60,000) in the same mass concentration range as that in Table 2, no gelation was observed under all conditions even after 1 hour at room temperature (Table 3). This is presumably because the molecular weight distribution of medical gelatin is relatively broad, and low molecular weight fragments that are not incorporated into the network structure have quenched the crosslinking reaction. On the other hand, the molecular weight distribution of CBE3 is relatively narrow, and it is presumed that a relatively efficient network formation could be achieved.

**[Table 3]**

| Precursor aqueous solution used for gel preparation and gelation possibility | | | | |
|---|---|---|---|---|
| | Crosslinking target | | Crosslinking agent | Gelation possibility |
| | CBE3 solution [g/L] | Medical gelatin solution [g/L] | PEG crosslinking agent aqueous solution [g/L] | |
| CBE3 + PEG crosslinking agent | 18.62 | - | 15 | Gelation possible |
| | 37.23 | - | 30 | Gelation possible |
| | 74.46 | - | 60 | Gelation possible |
| Medical gelatin + PEG crosslinking agent | - | 18.62 | 30 | Not gelated |
| | - | 37.23 | 30 | Not gelated |
| | - | 74.46 | 30 | Not gelated |
| | - | 37.23 | 60 | Not gelated |
| | - | 74.46 | 60 | Not gelated |
| | - | 148.92 | 60 | Not gelated |

### 2. Evaluation of gel characteristics

The gelation solution was poured into a silicone mold and allowed to be left overnight at room temperature. A compression test was performed on the prepared low/medium/high concentration gels, and the modulus of elasticity and the compressive rupture strength were evaluated. The results are shown in Table 4. To measure the compressive rupture strength, cylindrical low/medium/high concentration test pieces (ϕ = 10 mm, h = 5 mm) were compressed at a compression rate of 0.1 mm/sec using ElectroForce 5500 (TA Instruments), and data of displacement and force was obtained. From the obtained data, a stress-distortion curve was drawn, and the modulus of elasticity was calculated from the slope in the linear low distortion region. The stress at the time of rupture was defined as the compressive rupture strength.

**[Table 4]**

| Modulus of elasticity and compressive rupture strength of gel | | |
|---|---|---|
| Sample code | Modulus of elasticity [kPa] | Compressive rupture strength [MPa] |
| Low concentration | 3.45 | 0.06 |
| Medium concentration | 5.40 | 0.27 |
| High concentration | 10.39 | 6.11 |

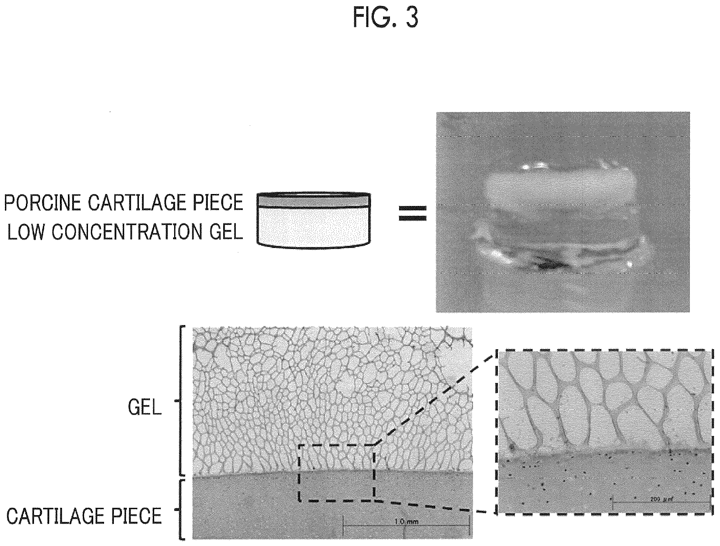

Cartilage pieces collected from the porcine joint were placed in a silicone mold, and the medium concentration gelation solution was poured on the cartilage pieces. After confirming the gelation and then confirming that the gel and the cartilage pieces had adhered macroscopically, the gel and the cartilage pieces were further incubated overnight in water at room temperature. The obtained sample was frozen and sliced and then stained with hematoxylin and eosin (Fig. 3). It has been found that the adhesion between the gel and the cartilage pieces is maintained in water in the micrometer scale even after a long period of time passed. This is presumably because a strong chemical bond is formed between the amino group derived from a protein on the surface of the cartilage piece and the unreacted PEG on the surface during gel formation.

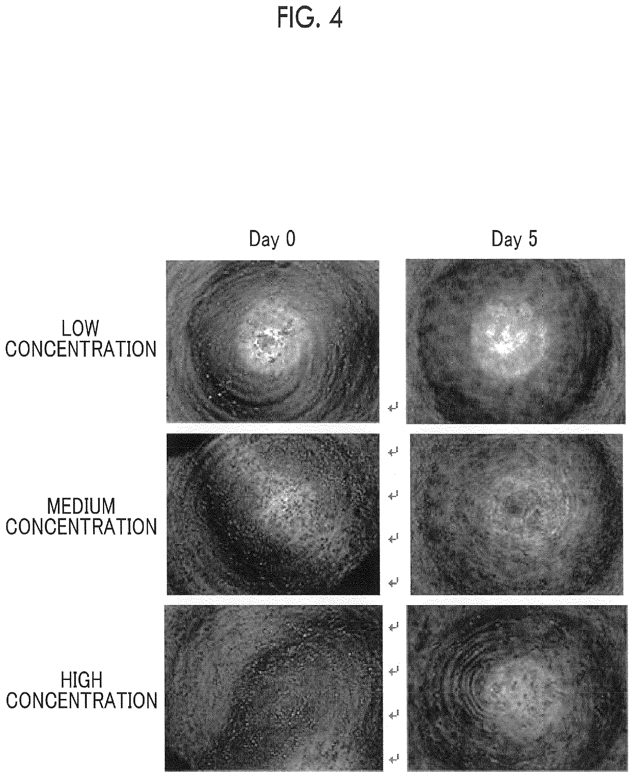

The gel was prepared on a 96-well plate and human bone marrow-derived mesenchymal stem cells were seeded (5 x 10⁴ cells/well). After 5 days, it was observed that the cells adhered and extended to the gel surface (Fig. 4). Considering that it is widely known that the gel consisting only of polyethylene glycol (PEG) is cell non-adhesive, it is presumed that the cell adhesiveness of the gel according to the embodiment of the present invention results from the exhibition of the cell adhesion ability of CBE3.

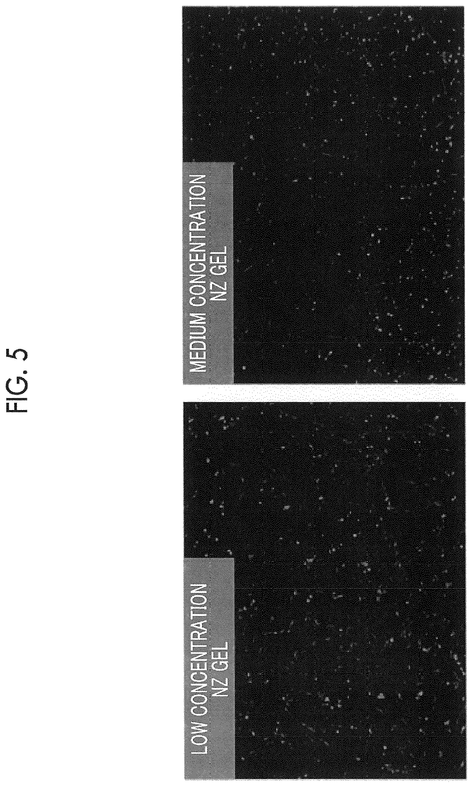

Human bone marrow-derived stem cells were contained in various gels, cultured for 21 days, and then a live/dead assay was performed. Many cells were alive in the low/medium concentration gels (Fig. 5).

Cellstain (registered trademark) Double Staining Kit (CS01, Dojindo Laboratories) for Live/dead assay was used according to the manufacturer's protocol and the stained cells were observed under a fluorescence microscope (BZ-X, KEYENCE CORPORATION).

### [Example 2]

### <Preparation of synovia-derived stem cell-containing gelation solution>

Synovia-derived stem cells (isolated from a Japanese white rabbit) (5.0 × 10⁵ cells, or 5.0 × 10⁶ cells) were mixed with 200 µL of the medium concentration gelation solution and a low concentration (1.0 × 10⁵ cells / 40 µL) cell-containing gelation solution and a high concentration (1.0 × 10⁶ cells / 40 µL) cell-containing gelation solution were prepared.

### <Evaluation of rabbit knee osteochondral defect model>

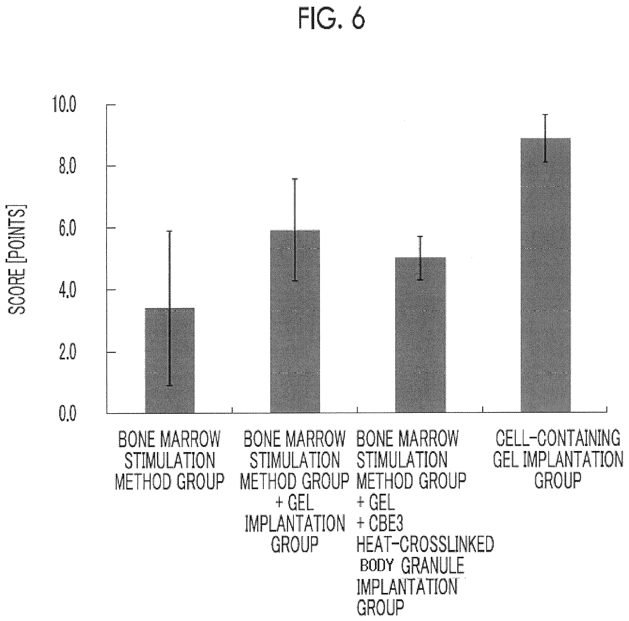

An osteochondral defect with a diameter of 5 mm and a depth of 2 mm was prepared in the affected part of a Japanese white rabbit (25 weeks old, weight: 3.5 to 4.1 kg), and bone marrow stimulation (BMS) was performed or not performed at a depth of 4 mm at 5 locations in the defect using Kirschner's wire (diameter: 0.8 mm, manufactured by MIZUHO Corporation), and then the medium concentration gelation solution was implanted. In addition to the bone marrow stimulation and the implantation of the medium concentration gelation solution, an experiment in which CBE3 heat-crosslinked body granules were implanted was carried out. In addition, after preparing the osteochondral defect in the same manner, an experiment in which the prepared low concentration cell-containing gelation solution was implanted and an experiment in which the high concentration cell-containing gelation solution was implanted were carried out without performing BMS. After allowing each gelation solution to be left for 10 minutes, the gelation was confirmed, and the affected part was closed. An autopsy was performed 12 weeks after the surgical operation, and the degree of cartilage restoration was scored based on histological evaluation (Tables 5 to 12 and Fig. 6). The cell-containing gel implantation group shown in Tables 8 and 12 is a low concentration cell-containing gel implantation group. In addition, the cell-containing gel implantation groups shown in Fig. 6 is a low concentration cell-containing gel implantation group.

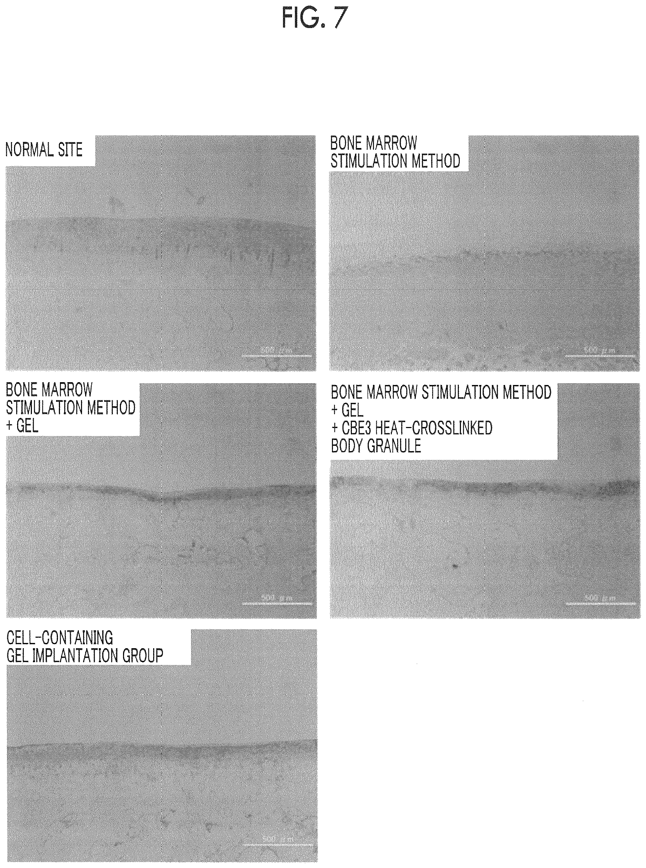

From the above results, it was suggested that a stable therapeutic effect is obtained since it has been shown that the variation is low and the average value of scores, which is an index of the therapeutic effect, is high in the BMS + medium concentration gelation solution implantation group as compared with BMS, which is the existing treatment. In addition, from the immunostaining image of type II collagen (Col II), which is a component of hyaline cartilage, a Col II-positive tissue was confirmed up to near the surface layer in the BMS + gelation solution implantation site in the present invention. This result indicates that the tissue has a structure similar to the normal site, suggesting that the tissue is a well restored cartilage tissue (Fig. 7). Even in the BMS + medium concentration gelation solution + CBE3 heat-crosslinked body granule implantation group, it was suggested that the restored tissue is better than that from BMS, which is the existing treatment, from the scoring of cartilage restoration and the immunostaining images. In addition, the cell-containing gel implantation group showed a high value of the scoring of cartilage restoration as compared with the BMS + medium concentration gelation solution implantation group and the BMS + medium concentration gelation solution + CBE3 heat-crosslinked body granule implantation group, suggesting that the cartilage restoring ability is high.

### <Processing of scoring value>

1. The average of the left/right end scores of each knee is scored as the treatment capability of each knee.
2. The average capability of each group is calculated and is defined as the capability value of each group.
3. The capability value of each group is plotted.
4. For each group, the standard deviation of the capability of each knee is calculated and added to the plot as an error bar.

### <Grade definition of scoring value>

(1) Horizontal direction (coverage (%))
   100%: 3
   ≥ 75%: 2
   ≥ 50%: 1
   < 50%: 0
(2) Matching of differentiated layer
   0 to 4+: 3
   5+: 2
   6+ to 10+: 1
   11+ or more: 0
(3) Thickness (degree of deviation from normal)
   Normal range: 3
   Thin/thick (mild): 2
   Thin/thick (medium): 1
   Thin/thick (high): 0

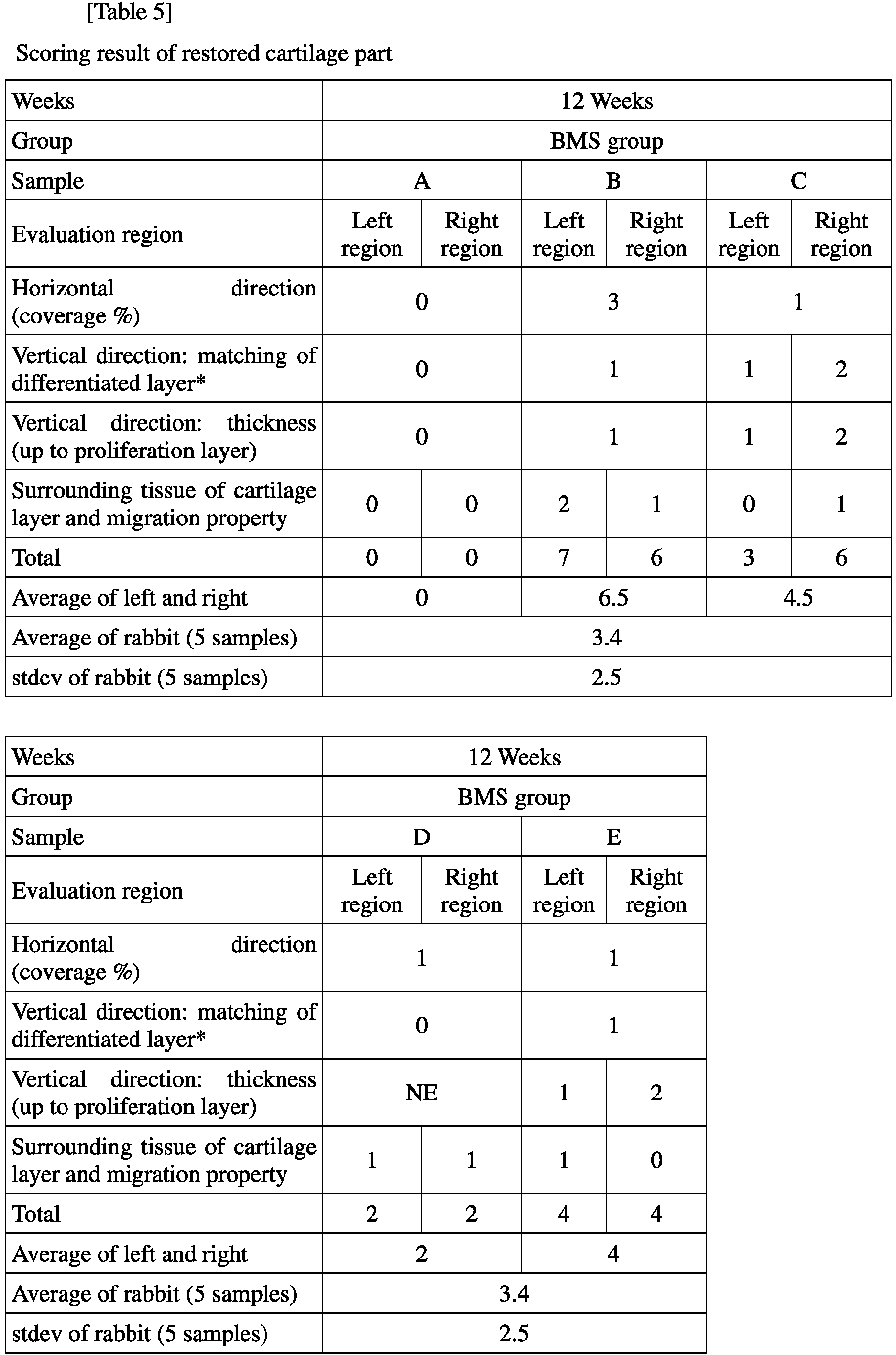

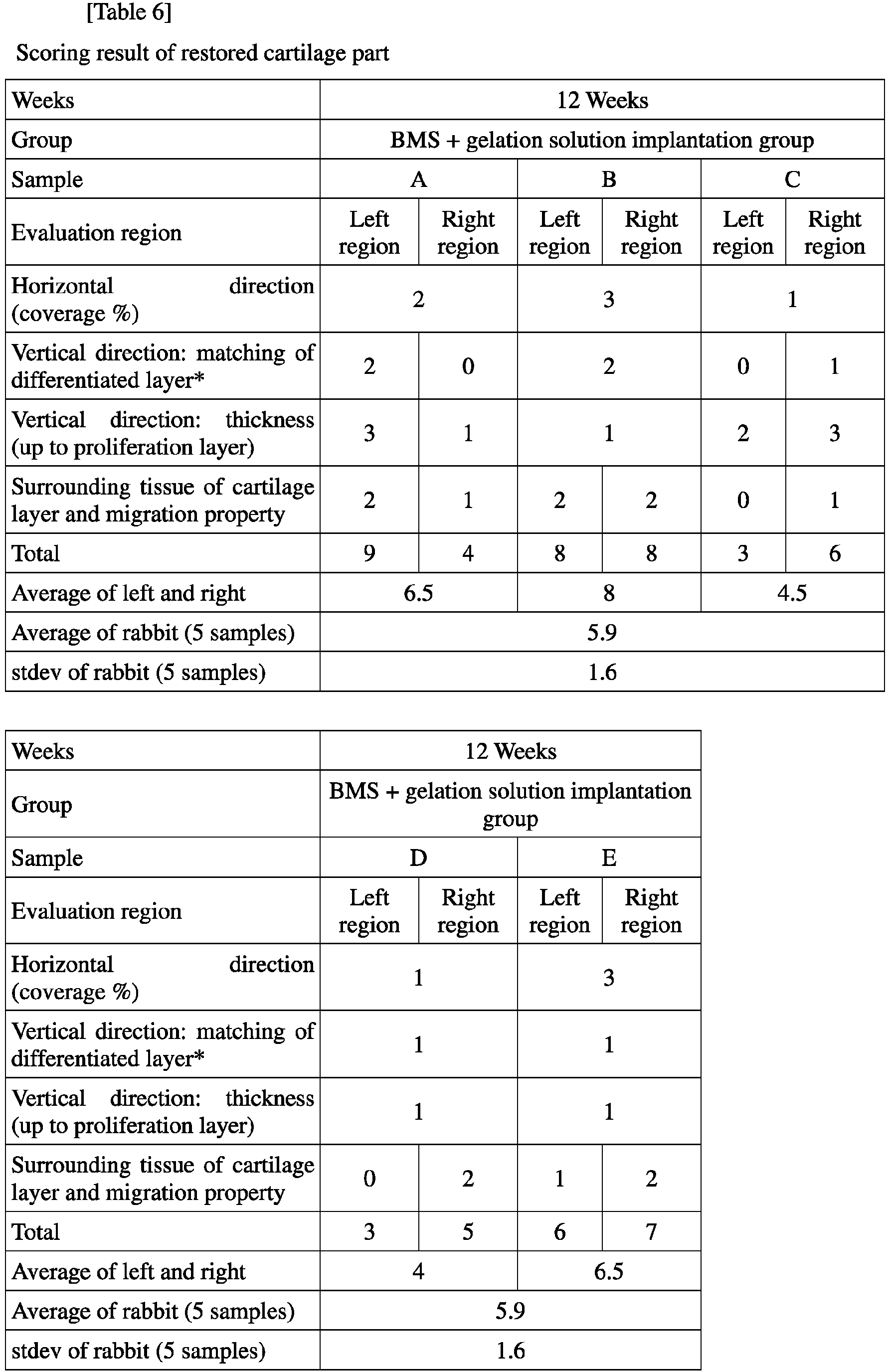

**[Table 7]**

| Scoring result of restored cartilage part | | | | |
|---|---|---|---|---|
| Weeks | 12 Weeks | | | |
| Group | BMS + gelation solution + CBE3 heat-crosslinked body granule implantation group | | | |
| Sample | A | | B | |
| Evaluation region | Left region | Right region | Left region | Right region |
| Horizontal direction (coverage %) | 2 | | 1 | |
| Vertical direction: matching of differentiated layer* | 1 | | 1 | 1 |
| Vertical direction: thickness (up to proliferation layer) | 1 | | 2 | 2 |
| Surrounding tissue of cartilage layer and migration property | 1 | 0 | 1 | 2 |
| Total | 5 | 4 | 5 | 6 |
| Average of left and right | 4.5 | | 5.5 | |
| Average of rabbit (2 samples) | 5.0 | | | |
| stdev of rabbit (2 samples) | 0.7 | | | |

**[Table 8]**

| Scoring result of restored cartilage part | | | | | | |
|---|---|---|---|---|---|---|
| Weeks | 12 Weeks | | | | | |
| Group | Cell-containing gelation solution implantation group | | | | | |
| Sample | A | | B | | C | |
| Evaluation region | Left region | Right region | Left region | Right region | Left region | Right region |
| Horizontal direction (coverage %) | 3 | | 3 | | 3 | |
| Vertical direction: matching of differentiated layer* | 2 | | 2 | | 1 | |
| Vertical direction: thickness (up to proliferation layer) | 3 | | 3 | | 2 | |
| Surrounding tissue of cartilage layer and migration property | 1 | 1 | 1 | 2 | 2 | 2 |
| Total | 9 | 9 | 9 | 10 | 8 | 8 |
| Average of left and right | 9 | | 9.5 | | 8 | |
| Average of rabbit (3 samples) | 8.8 | | | | | |
| stdev of rabbit (3 samples) | 0.8 | | | | | |

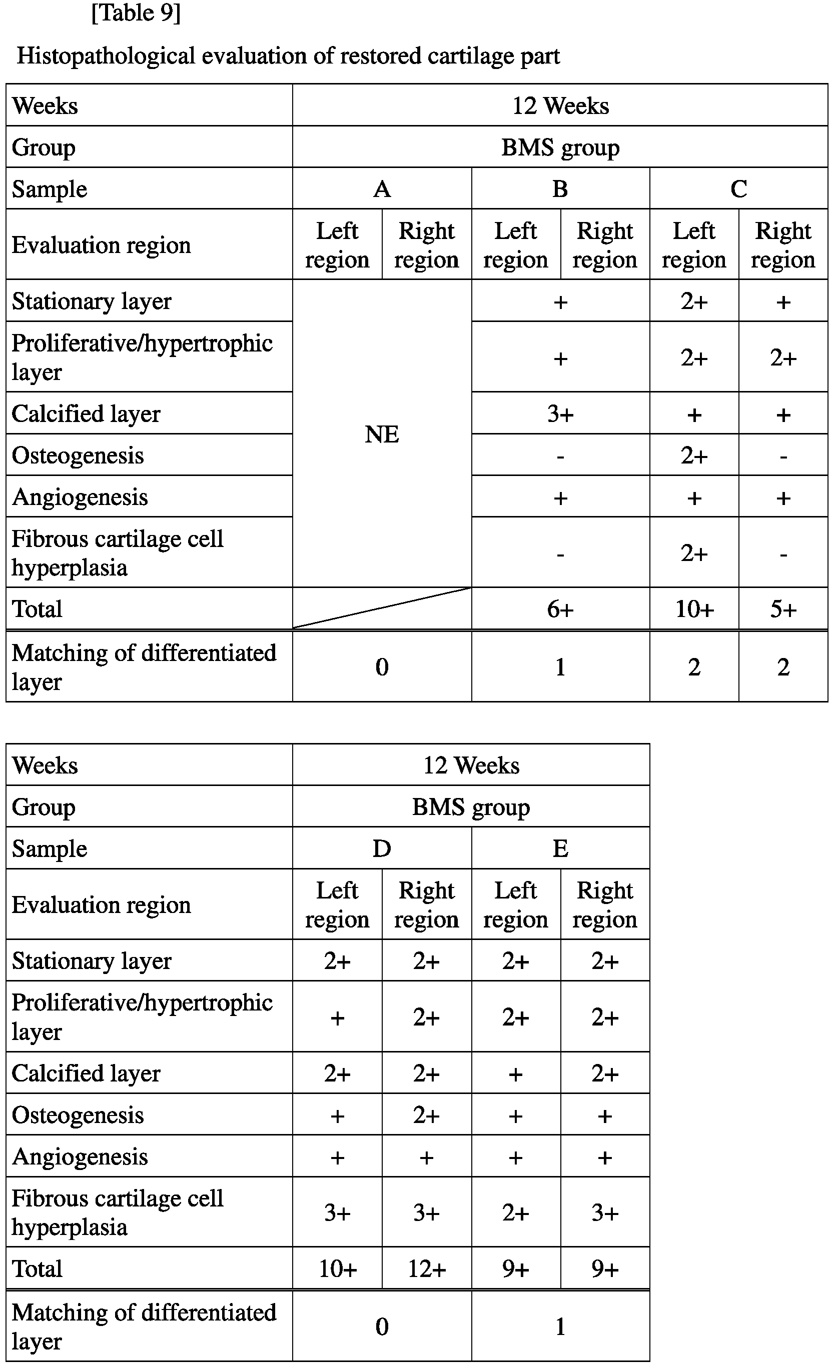

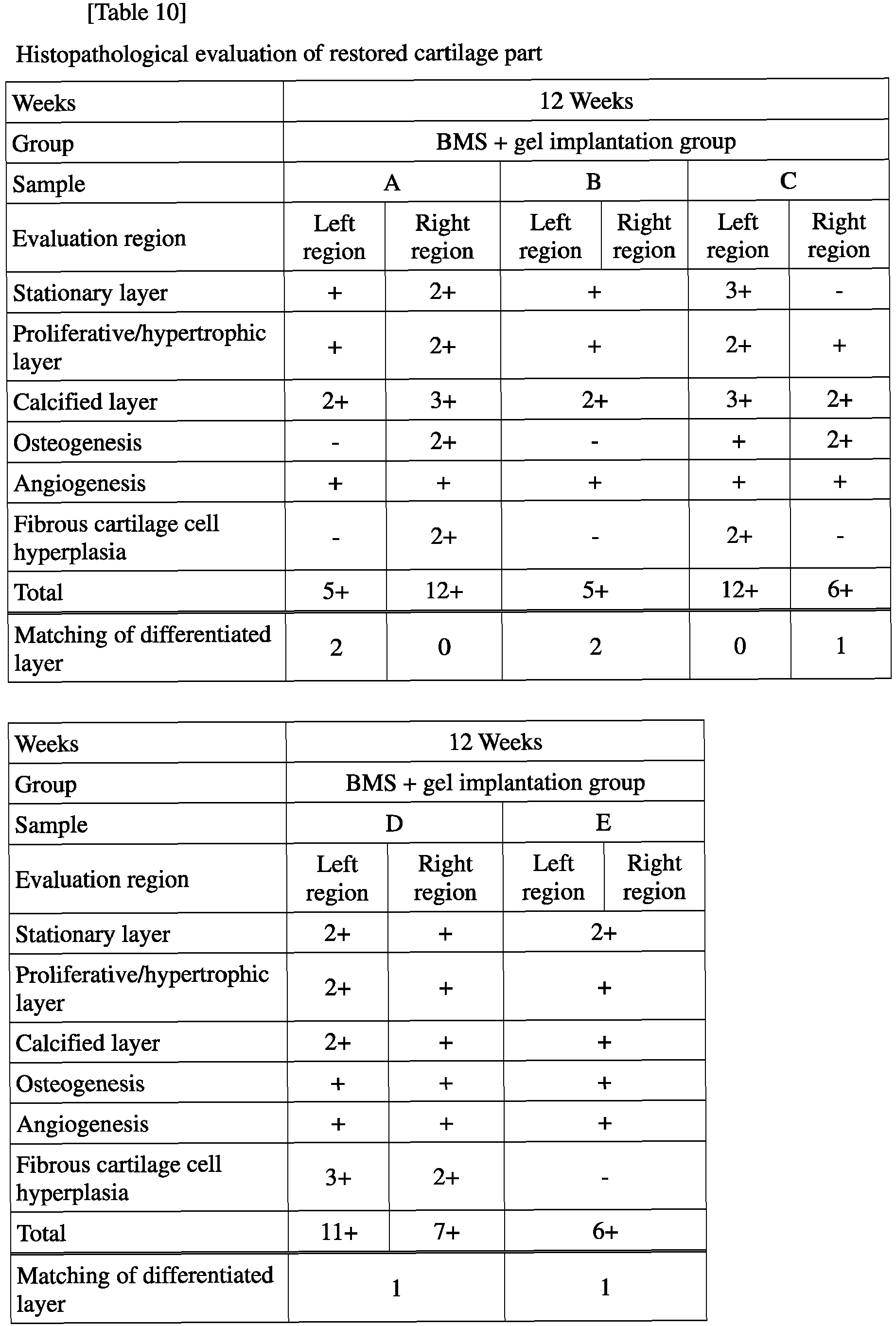

**[Table 11]**

| Histopathological evaluation of restored cartilage part | | | | |
|---|---|---|---|---|
| Weeks | 12 Weeks | | | |
| Group | BMS + gelation solution + CBE3 heat-crosslinked body granule implantation group | | | |
| Sample | A | | B | |
| Evaluation region | Left region | Right region | Left region | Right region |
| Stationary layer | + | | 2+ | + |
| Proliferative/hypertrophic layer | 2+ | | 2+ | 2+ |
| Calcified layer | 2+ | | 2+ | 2+ |
| Osteogenesis | - | | - | + |
| Angiogenesis | + | | + | + |
| Fibrous cartilage cell hyperplasia | + | | + | - |
| Total | 7+ | | 8+ | 7+ |
| Matching of differentiated layer | 1 | | 1 | 1 |

**[Table 12]**

| Histopathological evaluation of restored cartilage part | | | | | | |
|---|---|---|---|---|---|---|
| Weeks | 12 Weeks | | | | | |
| Group | Cell-containing gelation solution implantation group | | | | | |
| Sample | A | | B | | c | |
| Evaluation region | Left region | Right region | Left region | Right region | Left region | Right region |
| Stationary layer | + | | + | | + | |
| Proliferative/hypertrophic layer | + | | + | | 2+ | |
| Calcified layer | + | | + | | + | |
| Osteogenesis | + | | - | | 2+ | |
| Angiogenesis | + | | + | | + | |
| Fibrous cartilage cell hyperplasia | - | | + | | 2+ | |
| Total | 5+ | | 5+ | | 8+ | |
| Matching of differentiated layer | 2 | | 2 | | 1 | |

### [Sequence listing]

International application application based on the International Patent Cooperation Treaty 18F10540WUP19034390_14.app

## Claims

1. A gel forming kit comprising:
a crosslinking agent having at least two chains each of which includes a functional group capable of being covalently bonded to an amino group and includes a hydrophilic linking group; and
a recombinant peptide.

2. The gel forming kit according to claim 1, wherein the crosslinking agent and the recombinant peptide each have an injectable form.

3. The gel forming kit according to claim 1 or 2, wherein a combination of the crosslinking agent and the recombinant peptide exhibits a time-dependent gelation ability.

4. The gel forming kit according to any one of claims 1 to 3, wherein the crosslinking agent has a weight-average molecular weight of 5,000 to 30,000.

5. The gel forming kit according to any one of claims 1 to 4, wherein the chain which includes a functional group capable of being covalently bonded to an amino group and includes a hydrophilic linking group is represented by Formula 1,
Z-(A¹)_{w}-(B¹)ₓ-(C¹)_{y}- Formula 1
in the formula, Z is a functional group capable of being covalently bonded to an amino group, A¹ is a hydrophobic linking group, B¹ is a hydrophilic linking group, C¹ is a hydrophobic linking group, w is an integer of 1 or more, x is an integer of 1 or more, and y is an integer of 0 or more.

6. The gel forming kit according to any one of claims 1 to 5, wherein the functional group capable of being covalently bonded to an amino group is an isocyanate, an isothiocyanate, a sulfonyl chloride, an aldehyde, an acyl azide, an acid anhydride, an imide ester, an epoxide, or an active ester.

7. The gel forming kit according to any one of claims 1 to 6, wherein the hydrophilic linking group includes an ethylene oxide unit.

8. The gel forming kit according to any one of claims 1 to 7, wherein the recombinant peptide is a recombinant gelatin.

9. The gel forming kit according to claim 8, wherein the recombinant gelatin is represented by the following formula,
A-[(Gly-X-Y)n]m-B
in the formula, A represents any amino acid or amino acid sequence, B represents any amino acid or amino acid sequence, n pieces of X's each independently represent any one of amino acids, n pieces of Y's each independently represent any one of amino acids, n represents an integer of 3 to 100, m represents an integer of 2 to 10, and n pieces of Gly-X-Y's may be the same or different from each other.

10. The gel forming kit according to claim 8 or 9, wherein the recombinant gelatin is any one of the followings:
a peptide consisting of an amino acid sequence set forth in SEQ ID NO: 1;
a peptide consisting of an amino acid sequence which is obtained by deleting, substituting, or adding one or several amino acids in the amino acid sequence set forth in SEQ ID NO: 1, and having biocompatibility; or
a peptide consisting of an amino acid sequence which has 80% or more of a sequence identity with the amino acid sequence set forth in SEQ ID NO: 1, and having biocompatibility.

11. The gel forming kit according to any one of claims 1 to 10, wherein the gel forming kit is for use as a cartilage restoring agent.

12. The gel forming kit according to any one of claims 1 to 11, wherein the gel forming kit is for use as a cartilage restoring agent for promoting cartilage regeneration after performing bone marrow stimulation.

13. The gel forming kit according to any one of claims 1 to 12, further comprising a cell.

14. The gel forming kit according to claim 13, wherein the cell is a synovia-derived cell.

15. The gel forming kit according to claim 13 or 14, wherein a concentration of the cell with respect to a gel at a time of implantation is 1.0 × 10³ to 1.0 × 10⁷ cells / 40 µL.

16. A gel formed by crosslinking a recombinant peptide with a crosslinking agent having at least two chains each of which includes a functional group capable of being covalently bonded to an amino group and includes a hydrophilic linking group.

17. The gel according to claim 16, wherein the crosslinking agent has a weight-average molecular weight of 5,000 to 30,000.

18. The gel according to claim 16 or 17, wherein the chain which includes a functional group capable of being covalently bonded to an amino group and includes a hydrophilic linking group is represented by Formula 1,
Z-(A¹)_{w}-(B¹)ₓ-(C¹)_{y}- Formula 1
in the formula, Z is a functional group capable of being covalently bonded to an amino group, A¹ is a hydrophobic linking group, B¹ is a hydrophilic linking group, C¹ is a hydrophobic linking group, w is an integer of 1 or more, x is an integer of 1 or more, and y is an integer of 0 or more.

19. The gel according to any one of claims 16 to 18, wherein the functional group capable of being covalently bonded to an amino group is an isocyanate, an isothiocyanate, a sulfonyl chloride, an aldehyde, an acyl azide, an acid anhydride, an imide ester, an epoxide, or an active ester.

20. The gel according to any one of claims 16 to 19, wherein the hydrophilic linking group includes an ethylene oxide unit.

21. The gel according to any one of claims 16 to 20, wherein the recombinant peptide is a recombinant gelatin.

22. The gel according to claim 21, wherein the recombinant gelatin is represented by the following formula,
A-[(Gly-X-Y)n]m-B
in the formula, A represents any amino acid or amino acid sequence, B represents any amino acid or amino acid sequence, n pieces of X's each independently represent any one of amino acids, n pieces of Y's each independently represent any one of amino acids, n represents an integer of 3 to 100, m represents an integer of 2 to 10, and n pieces of Gly-X-Y's may be the same or different from each other.

23. The gel according to claim 21 or 22, wherein the recombinant gelatin is any one of the followings:
a peptide consisting of an amino acid sequence set forth in SEQ ID NO: 1;
a peptide consisting of an amino acid sequence which is obtained by deleting, substituting, or adding one or several amino acids in the amino acid sequence set forth in SEQ ID NO: 1 and, having biocompatibility; or
a peptide consisting of an amino acid sequence which has 80% or more of a sequence identity with the amino acid sequence set forth in SEQ ID NO: 1, and having biocompatibility.

24. The gel according to any one of claims 16 to 23, wherein a concentration of the crosslinking agent in the gel is 0.75% by mass to 3.0% by mass.

25. The gel according to any one of claims 16 to 24, wherein the gel is for use as a cartilage restoring agent.

26. The gel according to any one of claims 16 to 25, wherein the gel is for use as a cartilage restoring agent for promoting cartilage regeneration after performing bone marrow stimulation.

27. The gel according to any one of claims 16 to 26, further comprising a cell.

28. The gel according to claim 27, wherein the cell is a stem cell or a chondrocyte.

29. The gel according to claim 27 or 28, wherein the cell is a mesenchymal stem cell.

30. The gel according to any one of claims 27 to 29, wherein the cell is a synovia-derived cell.

31. A method for producing the gel according to any one of claims 16 to 30, the method comprising:
mixing a crosslinking agent having at least two chains each of which includes a functional group capable of being covalently bonded to an amino group and includes a hydrophilic linking group with a recombinant peptide and performing a crosslinking reaction.


SEQUENCE LISTING
<110>  FUJIFILM Corporation
<120>  Gel-forming kit, gel and method for producing gel
<130>\201@18F10540W1
<160>  10
<170>  PatentIn version 3.5
<210>  1
<211>  571
<212>  PRT
<213>  Recombinant
<400>  1
Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
            20                  25                  30
Arg Gly Asp Ala Gly Pro Lys Gly Ala Asp Gly Ala Pro Gly Ala Pro
        35                  40                  45
Gly Leu Gln Gly Met Pro Gly Glu Arg Gly Ala Ala Gly Leu Pro Gly
    50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
            100                 105                 110
Asp Ala Gly Pro Lys Gly Ala Asp Gly Ala Pro Gly Lys Asp Gly Val
        115                 120                 125
Arg Gly Leu Ala Gly Pro Ile Gly Pro Pro Gly Pro Ala Gly Ala Pro
    130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
                165                 170                 175
Asp Gly Ala Pro Gly Lys Asp Gly Val Arg Gly Leu Ala Gly Pro Pro
            180                 185                 190
Gly Ala Pro Gly Leu Gln Gly Ala Pro Gly Leu Gln Gly Met Pro Gly
        195                 200                 205
Glu Arg Gly Ala Ala Gly Leu Pro Gly Pro Lys Gly Glu Arg Gly Asp
    210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
                245                 250                 255
Glu Arg Gly Asp Ala Gly Pro Lys Gly Ala Asp Gly Ala Pro Gly Lys
            260                 265                 270
Asp Gly Val Arg Gly Leu Ala Gly Pro Ile Gly Pro Pro Gly Glu Arg
        275                 280                 285
Gly Ala Ala Gly Leu Pro Gly Pro Lys Gly Glu Arg Gly Asp Ala Gly
    290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
                325                 330                 335
Gly Leu Gln Gly Met Pro Gly Glu Arg Gly Ala Ala Gly Leu Pro Gly
            340                 345                 350
Pro Lys Gly Glu Arg Gly Asp Ala Gly Pro Lys Gly Ala Asp Gly Ala
        355                 360                 365
Pro Gly Lys Asp Gly Val Arg Gly Leu Ala Gly Pro Pro Gly Ala Pro
    370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro
                405                 410                 415

Lys Gly Ala Asp Gly Ala Pro Gly Ala Pro Gly Leu Gln Gly Met Pro
            420                 425                 430
Gly Glu Arg Gly Ala Ala Gly Leu Pro Gly Pro Lys Gly Glu Arg Gly
        435                 440                 445
Asp Ala Gly Pro Lys Gly Ala Asp Gly Ala Pro Gly Lys Asp Gly Val
    450                 455                 460
Arg Gly Leu Ala Gly Pro Ile Gly Pro Pro Gly Glu Arg Gly Ala Ala
465                 470                 475                 480
Gly Leu Pro Gly Pro Lys Gly Glu Arg Gly Asp Ala Gly Pro Lys Gly
                485                 490                 495
Ala Asp Gly Ala Pro Gly Lys Asp Gly Val Arg Gly Leu Ala Gly Pro
            500                 505                 510
Ile Gly Pro Pro Gly Pro Ala Gly Ala Pro Gly Ala Pro Gly Leu Gln
        515                 520                 525
Gly Met Pro Gly Glu Arg Gly Ala Ala Gly Leu Pro Gly Pro Lys Gly
    530                 535                 540
Glu Arg Gly Asp Ala Gly Pro Lys Gly Ala Asp Gly Ala Pro Gly Lys
545                 550                 555                 560
Asp Gly Val Arg Gly Leu Ala Gly Pro Pro Gly
                565                 570
<210>  2
<211>  4
<212>  PRT
<213>  Artificial Sequence
<220>
<223>  Description of Artificial Sequence: adhesive sequence
<400>  2
Arg Glu Asp Val
1
<210>  3
<211>  5
<212>  PRT
<213>  Artificial Sequence
<220>
<223>  Description of Artificial Sequence: adhesive sequence
<400>  3
Tyr Ile Gly Ser Arg
1               5
<210>  4
<211>  5
<212>  PRT
<213>  Artificial Sequence
<220>
<223>  Description of Artificial Sequence: adhesive sequence
<400>  4
Pro Asp Ser Gly Arg
1               5
<210>  5
<211>  7
<212>  PRT
<213>  Artificial Sequence
<220>
<223>  Description of Artificial Sequence: adhesive sequence
<400>  5
Arg Tyr Val Val Leu Pro Arg
1               5
<210>  6
<211>  6
<212>  PRT
<213>  Artificial Sequence
<220>
<223>  Description of Artificial Sequence: adhesive sequence
<400>  6

Leu Gly Thr Ile Pro Gly
1               5
<210>  7
<211>  10
<212>  PRT
<213>  Artificial Sequence
<220>
<223>  Description of Artificial Sequence: adhesive sequence
<400>  7
Arg Asn Ile Ala Glu Ile Ile Lys Asp Ile
1               5                   10
<210>  8
<211>  5
<212>  PRT
<213>  Artificial Sequence
<220>
<223>  Description of Artificial Sequence: adhesive sequence
<400>  8
Ile Lys Val Ala Val
1               5
<210>  9
<211>  4
<212>  PRT
<213>  Artificial Sequence
<220>
<223>  Description of Artificial Sequence: adhesive sequence
<400>  9
Asp Gly Glu Ala
1
<210>  10
<211>  4
<212>  PRT
<213>  Artificial Sequence
<220>
<223>  Description of Artificial Sequence: adhesive sequence
<400>  10
Glu Arg Gly Asp
1